# FASCICULE DE BREVET EUROPEEN

(11) **EP 2 723 197 B1**
(45) Date de publication et mention de la délivrance du brevet: **09.12.2015**
(21) Numéro de dépôt: 12730521.7
(22) Date de dépôt: 27.06.2012
(51) Int. Cl.: A23L 1/30, A61K 36/07, A23C 9/152, A61K 31/366

(54) **BOISSON LACTEE A BASE DE LOVASTATINE DE PLEUROTE**
MILCHGETRÄNK AUF DER BASIS VON AUSTERNPILZ-LOVASTATIN
MILK DRINK BASED ON OYSTER MUSHROOM LOVASTATIN

(30) Priorité: 27.06.2011 BE 201100386
(43) Date de publication de la demande: 30.04.2014
(73) Titulaire: Compagnie Des Brevets Nutraceutiques, 1380 Lasne (BE)
(72) Inventeur: MOTTE, François, B-1380 Lasne (BE); WYVEKENS, Guy, B-1380 Lasne (BE)
(74) Mandataire: Coulon, Ludivine
(86) Numéro de dépôt international: PCT/EP2012/062411
(87) Numéro de publication internationale: WO 2013/000934

(56) Documents cités:
- EP-A2- 1 908 358
- WO-A1-2006/071085
- JP-A- 2008 125 500
- BOBEK P, OZDIN L, KUNIAK L: "Effect of oyster mushroom (Pleurotus ostreatus) and its ethanolic extract in diet on absorption and turnover of cholesterol in hypercholesterolemic rat", NAHRUNG, vol. 40, no. 4, 1996, pages 222-224, XP002666226,
- DATABASE WPI Week 201054 Thomson Scientific, London, GB; AN 2010-K00048 XP002666227, LI S ET AL: "preparation of flavored yogurt...", & CN 101 755 909 A (FRUIT TREE RES INST TIANJIN FOREST IND) 30 juin 2010 (2010-06-30)
- ENDO A ET AL: "Dihydromonacolin L and monacolin X, new metabolites those inhibit cholesterol biosynthesis", JOURNAL OF ANTIBIOTICS, JAPAN ANTIBIOTICS RESEARCH ASSOCIATION, TOKYO, JP, vol. 38, no. 3, 1 mars 1985 (1985-03-01), pages 321-327, XP008085631, ISSN: 0021-8820

## Description

La présente invention se rapporte à une composition de complément alimentaire hypocholestérolémiante comprenant une base de composition et une quantité suffisante de monacoline pour obtenir un effet hypocholestérolémiant.

On connaît de nombreuses compositions hypocholestérolémiantes, parmi lesquelles se trouvent les compositions fermentées contenant des phytostérols. Ces compositions ont un effet sur la diminution de la captation du cholestérol ingurgité, l'empêchant de cette façon de pénétrer dans la circulation sanguine et d'y avoir un effet nocif. Toutefois, ces compositions hypocholestérolémiantes n'ont pas réellement d'effet curatif et sachant que près de 80 % du cholestérol dans le corps humain ne provient pas de source alimentaire mais bien de la synthèse de celui-ci par le foie, l'efficacité de telles compositions hypocholestérolémiantes apparaît limitée.

On connaît aussi l'effet de la monacoline K sur le taux de cholestérol. La monacoline K est un puissant inhibiteur de la HMG-CoA réductase. La biosynthèse du cholestérol est en effet contrôlée par un enzyme, la HMG-CoA. Si le taux de cholestérol d'un individu sain est bas, le foie produit la coenzyme HMG-CoA en haute quantité pour augmenter le taux de cholestérol dans l'individu sain. Si le taux de cholestérol chez cet individu sain est trop élevé, le foie inhibe ou réduit la production de la coenzyme pour, au contraire, réduire ou inhiber la production de cholestérol par le foie.

Dès lors, la monacoline K, en inhibant la coenzyme HMG-CoA permet de réduire le taux de cholestérol produit par un individu dont le taux de cholestérol est trop élevé.

Toutefois, la monacoline est accessible sur le marché des compléments alimentaires sous forme de levure de riz rouge et n'existe aujourd'hui qu'en gélule à concentration élevée, qui s'est avéré être une rare formulation possible étant donné le goût désagréable de cette dernière.

L'effet hypocholestérolémiant d'extrait de pleurote est connu de Bobek et al, Nahrung 1996; 40 (4): 222-224 (XP 002666226). Des produits laitiers comprenant des ingrédients obtenus à partir de champignons, dont la pleurote, sont décrits dans EP 1 908 358 et CN 101755909.

Il existe donc un besoin d'obtenir une composition de complément alimentaire liquide dont le goût est acceptable pour le consommateur, qui peut être consommée chaque jour tout en présentant un effet de réduction du taux de cholestérol produit par le foie.

Pour atteindre cet objectif, la composition selon l'invention est caractérisée en ce que la monacoline est de la lovastatine de pleurote et en ce que ladite base de composition est une base de lait liquide.

Il a en effet été montré que la lovastatine de pleurote présente des effets anti-cholestérol et que son mélange avec une base de lait liquide telle que du lait de vache entier, demi-écrémé, du lait écrémé, du pudding, du lait épaissi, un yaourt à boire, du lait de soja et analogue en fait une boisson au goût agréable, malgré le goût prononcé et particulier de la pleurote.

Avantageusement, la lovastatine de pleurote est de la lovastatine provenant du pleurote du genre *Pleurotus Ostreatus* qui est le genre de la famille du pleurote qui présente des taux élevés de lovastatine.

De préférence, ladite base de lait est du lait non fermenté. Dans tous les cas, la base de lait est mélangée à la lovastatine de pleurote et si elle est fermentée, elle ne l'est pas en présence de la lovastatine de pleurote, mais au préalable.

Selon une forme de réalisation préférentielle de l'invention, la composition de complément alimentaire hypocholestérolémiante comprend de 0,005 à 5 % en poids de poudre de pleurote par rapport au poids de la composition, sous forme d'un extrait standardisé, ladite poudre de pleurote comprenant ladite quantité suffisante de ladite lovastatine pour obtenir un effet hypocholestérolémiant.

De préférence, elle comprend de 0,005 à 0,5 % en poids de poudre de pleurote par rapport au poids de la composition, sous forme d'un extrait standardisé, ladite poudre de pleurote comprenant ladite quantité suffisante de ladite lovastatine pour obtenir un effet hypocholestérolémiant.

La poudre de pleurote présente de nombreux principes actifs tels que des antioxydants, des molécules à action sur le cholestérol, de nombreuses vitamines et oligoéléments ainsi que des protéines, qui sont forcément tous compatibles puisque présents dans le même organisme au départ, à savoir le pleurote et contrairement aux compléments à base de levure de riz rouge qui ne peuvent être mélangés à aucune autre substance active.

Parmi les molécules à action sur le cholestérol, on trouve des composés qui agissent à différentes étapes de la régulation du cholestérol sanguin. Les glucanes et la pectine sont présents dans le pleurote et sont des fibres solubles susceptibles de se lier aux acides biliaires et réduisent l'absorption du cholestérol dans les intestins. En plus de la lovastatine, on trouve également de la chitine qui est connue pour réduire le cholestérol chez les animaux. Dès lors, tous ces composés permettent d'obtenir une composition de complément alimentaire agréable au goût qui permet d'agir à de multiples niveaux de la fonction digestive pou réduire le taux de cholestérol.

De manière préférentielle, la composition comprend de 0,005 à 0,5 % de poudre de pleurote, sous forme d'un extrait standardisé, ladite poudre de pleurote comprenant ladite quantité suffisante pour obtenir un effet hypocholestérolémiant de ladite lovastatine.

Dans une forme de réalisation selon l'invention la composition comprend en outre un extrait de fruit, de préférence à hauteur de 0,3 % à 2,5 %, en particulier de 0,5 % à 1,5 % et de manière particulièrement préférentielle environ 1 % en volume par rapport au volume final de la composition.

Avantageusement, la composition selon l'invention comprend en outre un additif complémentaire choisi parmi des arômes, des édulcorants, des épaississants, des conservateurs.

D'autres formes de réalisation de la composition de complément alimentaire sont mentionnées dans les revendications annexées.

L'invention se rapporte également à une utilisation de poudre de pleurote dans une boisson lactée hypocholestérolémiante.

D'autres formes d'utilisation sont mentionnées dans les revendications annexées.

La présente invention va maintenant être décrite plus en détail au moyen d'un exemple de réalisation donné à titre non limitatif.

### Exemple

Le pleurote a été cultivé selon des normes précises afin de contenir une dose standard de Monacholine K (lovastatine). Ensuite les pleurotes récoltés sont chauffés et séchés avant d'être réduit en poudre.

On a ensuite ajouté la poudre de pleurote dans un lait fermenté sucré à raison de 250 mg/5 cl et l'ensemble a été mélangé jusqu'à obtenir une composition homogène.

La composition homogène est ensuite pasteurisée et conditionnée par dose de 5 cl.

Lors des tests de gouts, il a été observé de manière remarquable que le gout de l'extrait du pleurote dans le lait fermenté était plaisant sans laisser apparaître d'arrière-gout désagréable. De plus la texture de l'ensemble était satisfaisante.

## Revendications

1. Composition de complément alimentaire hypocholestérolémiante comprenant une base de composition et une quantité suffisante pour obtenir un effet hypocholestérolémiant de monacoline, dans laquelle ladite monacoline est de la lovastatine de pleurote et dans laquelle ladite base de composition est une base de lait liquide.

2. Composition hypocholestérolémiante selon la revendication 1, dans laquelle le pleurote est du genre Pleurotus Ostreatus.

3. Composition hypocholestérolémiante selon la revendication 1 ou la revendication 2, dans laquelle le lait écrémé de ladite base est du lait non fermenté.

4. Composition hypocholestérolémiante selon l'une quelconque des revendications 1 à 3, comprenant de 0,005 à 5 % en poids de poudre de pleurote par rapport au poids de la composition, sous forme d'un extrait standardisé, ladite poudre de pleurote comprenant ladite quantité suffisante pour obtenir un effet hypocholestérolémiant de ladite lovastatine.

5. Composition hypocholestérolémiante selon la revendication 4, comprenant de 0,005 à 0,5 % en poids de poudre de pleurote par rapport au poids de la composition, sous forme d'un extrait standardisé, ladite poudre de pleurote comprenant ladite quantité suffisante pour obtenir un effet hypocholestérolémiant de ladite lovastatine.

6. Composition selon l'une quelconque des revendications 1 à 5, comprenant en outre un extrait de fruit.

7. Composition selon l'une quelconque des revendications 1 à 6, comprenant en outre un additif complémentaire choisi parmi des arômes, des édulcorants, des épaississants, des conservateurs.

8. Composition hypocholestérolémiante selon l'une quelconque des revendications 1 à 7, dans laquelle ledit lait est du lait écrémé d'origine animale.

9. Composition hypocholestérolémiante selon l'une quelconque des revendications 1 à 8, sous la forme d'une boisson.

10. Utilisation de poudre de pleurote pour la préparation d'une boisson lactée hypocholestérolémiante.

## Patentansprüche

1. Mischung eines Nahrungsergänzungsmittels zur Senkung des Cholesterinspiegels, welches eine Mischungsgrundlage und eine ausreichende Menge von Monacolin enthält, um eine den Cholesterinspiegel senkende Wirkung zu erreichen, wobei das erwähnte Monacolin Austernpilz-Lovastatin ist und wobei die erwähnte Mischungsgrundlage eine Grundlage flüssiger Milch ist.

2. Mischung zur Senkung des Cholesterinspiegels nach Anspruch 1, in der der Austernpilz von der Spezies Pleurotus ostreatus ist.

3. Mischung zur Senkung des Cholesterinspiegels nach Anspruch 1 oder Anspruch 2, in der die Magermilch der erwähnten Grundlage nicht fermentierte Milch ist.

4. Mischung zur Senkung des Cholesterinspiegels nach irgendeinem der Ansprüche 1 bis 3, welche im Verhältnis zum Gewicht der Mischung 0,005 bis 5 Gew.% Austernpilzpulver in Form eines standardisierten Extraktes enthält, wobei das erwähnte Austernpilzpulver die erwähnte ausreichende Menge des erwähnten Lovastatins enthält, um eine den Cholesterinspiegel senkende Wirkung zu erreichen.

5. Mischung zur Senkung des Cholesterinspiegels nach Anspruch 4, welche im Verhältnis zum Gewicht der Mischung 0,005 bis 0,5 Gew.% Austernpilzpulver in Form eines standardisierten Extraktes enthält, wobei das erwähnte Austernpilzpulver die erwähnte ausreichende Menge des erwähnten Lovastatins enthält, um eine den Cholesterinspiegel senkende Wirkung zu erreichen.

6. Mischung nach irgendeinem der Ansprüche 1 bis 5, welche darüber hinaus einen Fruchtextrakt enthält.

7. Mischung nach irgendeinem der Ansprüche 1 bis 6, welche darüber hinaus einen ergänzenden Zusatzstoff gewählt aus Aromastoffen, Süßstoffen, Verdickungsmitteln, Konservierungsmitteln enthält.

8. Mischung zur Senkung des Cholesterinspiegels nach irgendeinem der Ansprüche 1 bis 7, in der die erwähnte Milch Magermilch tierischen Ursprungs ist.

9. Mischung zur Senkung des Cholesterinspiegels nach irgendeinem der Ansprüche 1 bis 8 in Form eines Getränks.

10. Verwendung von Austernpilzpulver für die Zubereitung eines Milchgetränks zur Senkung des Cholesterinspiegels.

## Claims

1. A cholesterol-lowering food supplement composition comprising a composition base and a sufficient amount for obtaining a monacolin cholesterol-lowering effect, wherein said monacolin is oyster mushroom lovastatin and wherein said composition base is a liquid milk base.

2. The cholesterol-lowering composition according to claim 1, wherein the oyster mushroom is of the *Pleurotus Ostreatus* genus.

3. The cholesterol-lowering composition according to claim 1 or claim 2, wherein the skimmed milk of said base is non-fermented milk.

4. The cholesterol-lowering composition according to any of claims 1 to 3, comprising from 0.005 to 5% by weight of oyster mushroom powder based on the weight of the composition, as a standardized extract, said oyster mushroom powder comprising said sufficient amount for obtaining a cholesterol-lowering effect of said lovastatin.

5. The cholesterol-lowering composition according to claim 4, comprising from 0.005 to 5% by weight of oyster mushroom powder based on the weight of the composition, as a standardized extract, said oyster mushroom powder comprising said sufficient amount for obtaining a cholesterol-lowering effect of said lovastatin.

6. The composition according to any of claims 1 to 5, further comprising a fruit extract.

7. The composition according to any of claims 1 to 6, further comprising a complementary additive selected from among flavors, sweeteners, thickeners, preservatives.

8. The cholesterol-lowering composition according to any of claims 1 to 7, wherein said milk is skimmed milk of animal origin.

9. The cholesterol-lowering composition according to any of claims 1 to 8, as a drink.

10. The use of oyster mushroom powder for preparing a cholesterol-lowering milk drink.
